(19) Europäisches Patentamt
European Patent Office
Office européen des brevets

(11) EP 1 837 353 A1

(12) DEMANDE DE BREVET EUROPEEN

(43) Date de publication:
**26.09.2007 Bulletin 2007/39**

(51) Int Cl.:
***C08G 18/08*** *(2006.01)* ***C08G 18/69*** *(2006.01)*
***A61K 8/87*** *(2006.01)* ***A61Q 5/06*** *(2006.01)*

(21) Numéro de dépôt: **07101734.7**

(22) Date de dépôt: **05.02.2007**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **20.03.2006 FR 0650951**

(71) Demandeur: **L'ORÉAL**
**75008 Paris (FR)**

(72) Inventeurs:
- **Mougin, Nathalie**
**75011, Paris (FR)**
- **Schultze, Xavier**
**77340, Pontault-Combault (FR)**

(74) Mandataire: **Dodin, Catherine**
**L'OREAL - D.I.P.I.**
**25-29 Quai Aulagnier**
**92600 Asnières (FR)**

# (54) Nouveaux polyuréthanes, compositions les comprenant et procédé de traitement cosmétique

(57) La présente invention concerne de nouveaux polyuréthanes constitués essentiellement de motifs cationiques, de diisocyanates et de motifs non ioniques dérivés de polyoléfines comprenant au moins 10% en mole d'unités comprenant au moins une double liaison carbone-carbone résiduelle.

L'invention concerne également les compositions cosmétiques ou pharmaceutiques comprenant lesdits polyuréthanes, ainsi qu'un procédé de traitement cosmétique, notamment de mise en forme et/ou de maintien des cheveux, comprenant l'application d'une telle composition.

EP 1 837 353 A1

## Description

La présente invention concerne des nouveaux polymères, notamment polyuréthanes, cationiques et à caractère élastique ainsi que leur utilisation dans des compositions cosmétiques ou pharmaceutiques, et les compositions cosmétiques ou pharmaceutiques ainsi obtenues.

La formation de dépôts et de films à propriétés élastiques fait depuis toujours l'objet d'importantes recherches en cosmétique. En effet, la plupart des zones du corps humain susceptibles de recevoir des dépôts cosmétiques, telles que la peau, les lèvres, les cheveux, les cils et les ongles, sont soumises à des déformations et contraintes mécaniques importantes. Les films et dépôts cosmétiques doivent pouvoir résister à ces contraintes et suivre ces déformations sans se casser.

L'utilisation de polyuréthanes en cosmétique est connue depuis longtemps et est décrite par exemple dans les brevets WO94/13724 et EP619111.

Les polyuréthanes décrits dans ces documents présentent cependant des températures de transition vitreuse (Tg) supérieures à la température ambiante (20°C), ceci signifie qu'à la température ambiante, ils sont à l'état vitreux et forment des films cassants, inacceptables pour une application cosmétique.

Il existe certes des polymères physiologiquement acceptables présentant des températures de transition vitreuse basses, comme par exemple les polymères acryliques, mais ces polymères forment généralement des dépôts très collants, ce qui présente un inconvénient dans la plupart des applications cosmétiques.

On connaît par ailleurs de la demande WO02/32978 des polyuréthanes physiologiquement acceptables formant des films non collants, non cassants et capables de déformations plastiques et élastiques. Ces propriétés viscoélastiques intéressantes sont dues à la présence, dans le polymère, de longs motifs macromoléculaires ayant une température de transition vitreuse relativement faible et qui de ce fait, à température ambiante, ne se trouvent pas à l'état vitreux.

Notamment, les polymères décrits comportent des motifs macromoléculaires de type polyéther, en particulier polytétraméthylèneoxyde (PTMO), ou encore de type copolymère (éthylène-butylène).

On peut encore citer le brevet US3388087 qui décrit des polyuréthanes comportant des motifs polypropylène glycol éther ou polybutylène glycol éther.

Ces polymères comportent des motifs hygroscopiques, ce qui nuit à la qualité cosmétique du polymère appliqué sur le cheveu; le polymère est alors trop hydrophile, il présente du collant et est peu résistant à l'eau.

Le but de la présente invention est de proposer des polymères, notamment polyuréthanes, physiologiquement acceptables et présentant des propriétés filmogènes et viscoélastiques améliorées.

Il est également recherché des polymères, notamment de type polyuréthane, permettant d'obtenir une composition cosmétique présentant une meilleure tenue à l'humidité et/ou à l'eau, et donc une tenue dans le temps du caractère coiffant suffisante.

Ce but est notamment atteint grâce à l'utilisation de polymères bien particuliers, obtenus à partir de polyoléfines, et qui présentent un caractère hydrophobe tel que les produits de coiffage, notamment les gels, les sprays et/ou les mousses capillaires, les comprenant permettent d'apporter du maintien à la chevelure, tout en conservant une tenue dans le temps de ce coiffant suffisante.

Ceci est particulièrement avantageux lors de la réalisation de coiffure avec 'méchés' (épi).

La présente invention a pour objet un polyuréthane constitué essentiellement :

(a1) d'au moins un motif cationique ou cationisable dérivé d'au moins une amine tertiaire ou quaternaire présentant au moins deux fonctions réactives à hydrogène labile,

(a2) d'au moins un motif non ionique dérivé d'au moins une polyoléfine présentant au moins deux fonctions réactives à hydrogène labile, ladite polyoléfine comprenant au moins 10% en mole d'unités comprenant au moins une double liaison C=C (carbone-carbone), par rapport à la totalité des unités formant ladite polyoléfine;

(b) d'au moins un motif dérivé d'un composé comportant au moins deux fonctions isocyanate.

L'invention concerne également une composition comprenant, dans un milieu physiologiquement acceptable, au moins un tel polyuréthane.

Le polymère selon l'invention présente de préférence un caractère élastique; on entend par là que ledit polymère est un matériau macromoléculaire qui retourne rapidement à sa forme et à ses dimensions initiales après cessation d'une contrainte faible ayant produit une déformation importante.

Le polymère selon l'invention, grâce à son caractère cationique/cationisable, présente par ailleurs l'avantage d'avoir une excellente affinité pour les substrats kératiniques tels que les cheveux, les ongles et la couche cornée de l'épiderme auxquels la kératine confère une charge négative.

L'utilisation du polymère selon l'invention dans des laques et des compositions de coiffage permet d'améliorer la souplesse de la coiffure, c'est-à-dire d'obtenir une tenue des cheveux plus naturelle et plus durable que celle obtenue avec des polymères fixants de l'art antérieur.

Enfin, les polymères ne sont pas collants, ce qui facilitent leur utilisation en cosmétique.
De plus, les compositions comprenant un polymère selon l'invention présentent une meilleure tenue à l'humidité grâce à l'utilisation de copolymères d'oléfines qui présentent un caractère hydrophobe.

Le polymère selon l'invention peut être obtenu par polycondensation des composés portant des fonctions réactives à hydrogène labile avec des composés comportant au moins deux fonctions isocyanate;
On entend par fonctions réactives à hydrogène labile des fonctions capables, après départ d'un atome d'hydrogène, de former des liaisons covalentes avec les fonctions isocyanate des composés comportant au moins deux fonctions isocyanate. On peut citer à titre d'exemple de telles fonctions les groupes hydroxyle, amine primaire ou amine secondaire, ou encore les groupes thiol.
Selon la nature des fonctions réactives portant l'hydrogène labile (-OH, $-NH_2$, - NHR ou -SH), la polycondensation conduit à, respectivement, des polyuréthanes, des polyurées ou des polythiouréthanes. Ainsi, les polymères utilisés dans l'invention peuvent être des copolymères uréthane/urée et/ou thiouréthane. Tous ces polymères sont regroupés dans la présente demande, par souci de simplification, sous le terme de polyuréthanes.

Le polyuréthane cationique ou cationisable selon l'invention comprend donc au moins un motif cationique ou cationisable (a1) résultant d'au moins une amine tertiaire ou quaternaire présentant au moins deux fonctions réactives à hydrogène labile.
Par motif cationique ou cationisable, on entend au sens de la présente invention tout motif qui, soit par sa nature chimique propre, soit en fonction du milieu et/ou du pH dans lequel il se trouve, sera sous forme cationique.
L'amine tertiaire est de préférence protonable à un pH choisi entre pH 1 et pH 12. Par "protonable", on entend que ladite fonction amine tertiaire peut être neutralisée au moins partiellement par un agent neutralisant ou par en fonction du milieu dans lequel il est formulé.
Lorsque les amines tertiaires ou quaternaires formant les motifs (a1) portent plus de deux fonctions à hydrogène labile, le polyuréthane obtenu présente une structure ramifiée.
Toutefois, de préférence, les amines tertiaires ou quaternaires formant les motifs (a1) ne présentent que deux fonctions réactives à hydrogène labile et les polyuréthanes obtenus par polycondensation ont par conséquent une structure essentiellement linéaire.
Il est bien entendu également possible d'utiliser un mélange d'amines difonctionnelles contenant, ou non, une faible proportion d'amines portant plus de deux fonctions réactives à hydrogène labile.

Les amines tertiaires ou quaternaires formant les motifs cationiques ou cationisables (a1) sont de préférence choisies parmi les composés correspondant à l'une des formules suivantes :

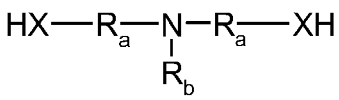

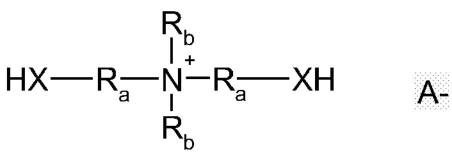

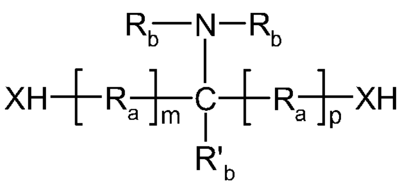

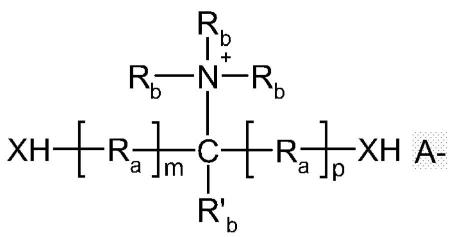

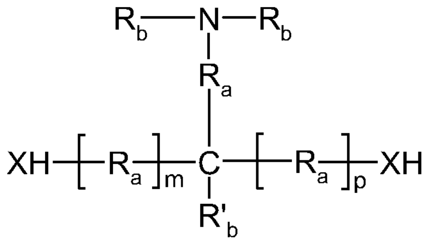

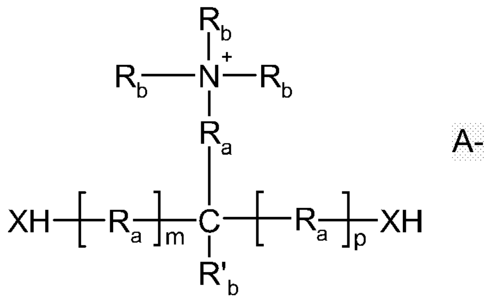

dans lesquelles :

- chaque $R_a$, indépendamment les uns des autres, représente un groupe divalent alkylène en $C_1$-$C_6$, linéaire ou ramifié, ou bien cycloalkylène en $C_3$-$C_6$ ou arylène,

ou leurs mélanges; ces groupes pouvant être substitués par un ou plusieurs atomes d'halogène et/ou comporter un ou plusieurs hétéroatomes choisis parmi O, N, P et S,

- chaque $R_b$ représente indépendamment les uns des autres, un groupe alkyle linéaire ou ramifié, en $C_1$-$C_6$, ou bien cycloalkyle en $C_3$-$C_6$ ou encore aryle, ou leurs mélanges; ces groupes pouvant être substitués par un ou plusieurs atomes d'halogène et/ou comporter un ou plusieurs hétéroatomes choisis parmi O, N, P et S,
- chaque R'b représente H ou un groupe alkyle linéaire ou ramifié, en $C_1$-$C_6$, ou bien cycloalkyle en $C_3$-$C_6$ ou encore aryle, ou leurs mélanges; ces groupes pouvant être substitués par un ou plusieurs atomes d'halogène et/ou comporter un ou plusieurs hétéroatomes choisis parmi O, N, P et S,
- m et p sont, indépendamment l'un de l'autre, égaux à 0 ou 1; de préférence m= 1 et p= 1;
- chaque X représente, indépendamment les uns des autres, un atome d'oxygène ou de soufre, ou un groupe NH ou $NR_c$, où $R_c$ représente un groupe alkyle en $C_1$-$C_6$, et
- $A^-$ représente un contre-ion physiologiquement acceptable, et notamment un halogénure tel que chlorure ou bromure.

De préférence, les amines sont de formule :

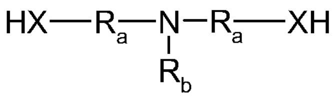

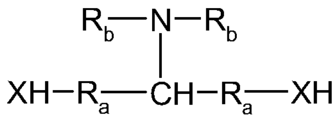

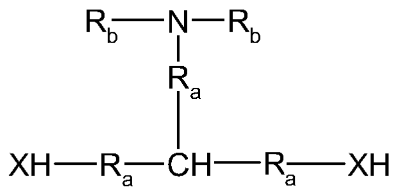

De préférence Ra est un groupe divalent alkylène en $C_1$-$C_6$, linéaire ou ramifié, notamment méthylène ou éthylène.
De préférence, Rb est un groupe alkyle linéaire ou ramifié, en $C_1$-$C_6$, notamment un groupe méthyle, éthyle, n-butyle, isobutyle ou tert-butyle.
De préférence, X = O.

Encore plus préférentiellement, les amines sont de formule :

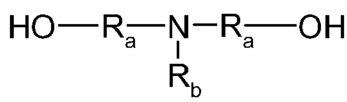

dans laquelle Ra est un groupe divalent alkylène en $C_1$-$C_6$, linéaire ou ramifié, notamment méthylène ou éthylène; et Rb est un groupe alkyle linéaire ou ramifié, en $C_1$-$C_6$, notamment un groupe méthyle, éthyle, n-butyle, isobutyle ou tert-butyle.

On peut citer à titre d'amines tertiaires particulièrement préférées, la N-méthyldiéthanolamine et la N-tert-butyldiéthanolamine.

Les amines tertiaires, protonables, peuvent être neutralisées, totalement ou partiellement, par un agent neutralisant, de type acide organique comprenant au moins une fonction acide carboxylique, sulfonique et/ou phosphonique ou par un acide minéral. On peut citer à titre d'exemple d'acides préférés l'acide chlorhydrique, l'acide sulfurique, l'acide acétique, l'acide propionique, l'acide citrique, l'acide gluconique, l'acide tartrique, l'acide lactique, l'acide phosphorique, l'acide benzoïque, l'acide stéarique, l'acide oléique, l'acide 2-éthylcaproïque, l'acide béhénique, le chlorhydrate de bétaïne, et leur mélange.

Le polyuréthane cationique selon l'invention comprend également au moins un motif non ionique (a2) résultant d'au moins une polyoléfine présentant au moins deux fonctions réactives à hydrogène labile, ladite polyoléfine comprenant au moins 10% en mole d'unités comprenant au moins une double liaison C=C (résiduelle), par rapport à la totalité des unités formant ladite polyoléfine.

De préférence, la ou les polyoléfines sont non ioniques.

De préférence, les fonctions réactives à hydrogène labile sont situées aux extrémités de la polyoléfine. Notamment, lesdites fonctions réactives à hydrogène labile sont des hydroxydes. De façon préférentielle, le nombre de motifs hydroxyde est proche de, voire égal à, 2.

De préférence encore, la ou les polyoléfine(s) formant le motif (a2) est choisie parmi les homopolymères et/ou copolymères d'oléfines, portant à leurs extrémités des fonctions réactives à hydrogène labile et ayant une température de transition vitreuse (Tg), mesurée par analyse enthalpique différentielle (DSC, differential scanning calorimetry) selon la norme ASTM D3418-97, inférieure à 10°C.

Le polyuréthanne selon l'invention peut comprendre plusieurs motifs (a2) résultant de plusieurs polyoléfines, identiques ou différentes (mélanges de polyoléfines); toutefois, dans ce cas, chacune des polyoléfines comprend au moins 10% molaire d'unités comprenant au moins une double liaison C=C.

On entend par 'unité comprenant une double liaison C=C', une unité qui comprend au moins une double liaison C=C résiduelle, de préférence une seule double liaison; il peut s'agir, par exemple d'une unité issue de la polymérisation d'une unité butadiène ou isoprène, toutes formes isomériques incluses (cis ou trans, 1,2 ou 1,4).

La polyoléfine selon l'invention peut être un homopolymère d'oléfines. On peut par exemple citer les homopolymères de 1,2-butadiène, de 1,4-butadiène ou d'isoprène, et notamment :

- les 1,4-polybutadiène, sous leurs formes cis et trans, :

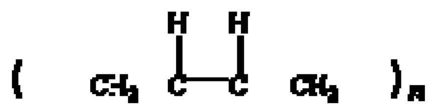

- les 1,2-polybutadiene : -[CH2-CH(CH=CH2)-]n

- les poly(cis-1,4-isoprene):

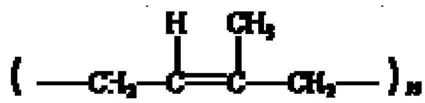

- les poly(trans-1,4-isoprene) :

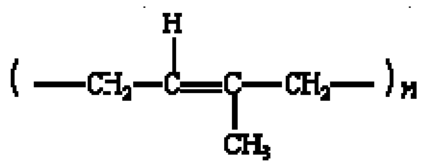

La polyoléfine selon l'invention peut également être un copolymère de différentes oléfines (copolymère d'oléfines), sous réserve que la polyoléfine finale comprenne au moins 10% molaire d'unités comprenant au moins une double liaison C=C.

Dans un premier mode de réalisation, ladite polyoléfine peut être exclusivement constituée d'unités comprenant

au moins une double liaison C=C. On peut par exemple citer les copolymères, notamment statistiques, comprenant des unités 1,2-butadiène et/ou des unités 1,4-butadiène dans ses formes cis et/ou trans, et/ou des unités isoprène, notamment cis-1,4-isoprène et trans-1,4-isoprène, en mélange. On peut notamment citer les copolymères statistiques (1,2-butadiène/1,4-butadiène).

De préférence, les polyoléfines peuvent être statistiques et à terminaisons hydroxy et répondre à la structure suivante:

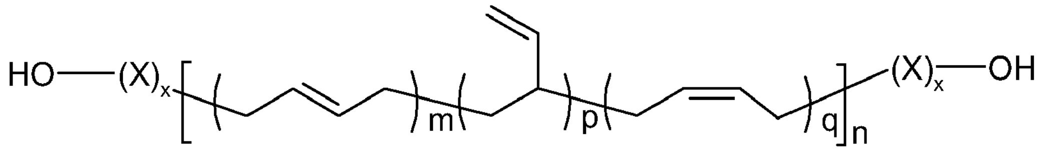

dans laquelle :

m, n et p étant des fractions molaires comprises entre 0 et 1, et m+n+p=1;

notamment m compris entre 0,1 et 0,8, voire 0,15 et 0,7; p compris entre 0,1 et 0,8, voire 0,15 et 0,7; et q compris entre 0,05 et 0,5, voire 0,1 et 0,4;

n est un entier compris entre 10 et 100, notamment entre 15 et 50;

x = 0 ou 1, et

X = radical carboné divalent, notamment alkylène, linéaire, cyclique ou ramifié, comprenant 1 à 10 atomes de carbone; notamment méthylène, éthylène, propylène ou isopropylène.

Elles peuvent présenter, de préférence, une masse moléculaire moyenne en nombre, Mn, comprise entre 400 et 50000, de préférence entre 500 et 30000, mieux entre 1000 et 15000, et encore mieux entre 1500 et 12000.

Plus particulièrement, on peut citer :

- les polybutadiènes à terminaisons hydroxy, tels que les polymères de structure :

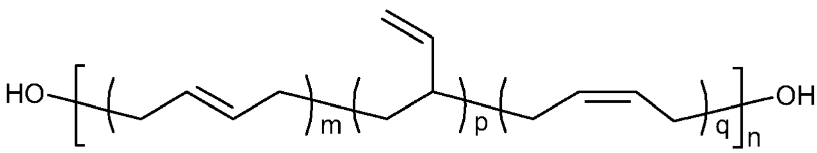

  avec m = 0,6, p = 0,2 et q= 0,2 (fractions molaires) et n= 25.

  On peut en particulier citer les produits commerciaux 'Poly bd R20LM' et 'Poly bd R45HTLO' de Sartomer;

- les polybutadiènes à terminaisons primaires hydroxy, tels que les polymères pouvant être représentés par la structure suivante :

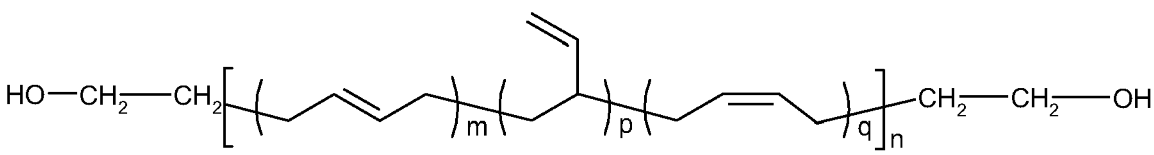

  qui sont des copolymères statistiques notamment de 1,4-cis-butadiène et de 1,4-trans-butadiène, avec m=0,17, p=0,65 et q=0,18 (fractions molaires) et n est tel que le poids moléculaire moyen en nombre Mn est compris entre 1000 et 10000, notamment entre 2000 et 6000 ($g.mol^{-1}$).

  On peut en particulier citer les produits commerciaux KRASOL LBH-P 2000, 3000

ou 5000 de Sartomer.

- les polybutadiènes à terminaisons secondaires hydroxy, tels que les polymères pouvant être représentés par la

structure suivante :

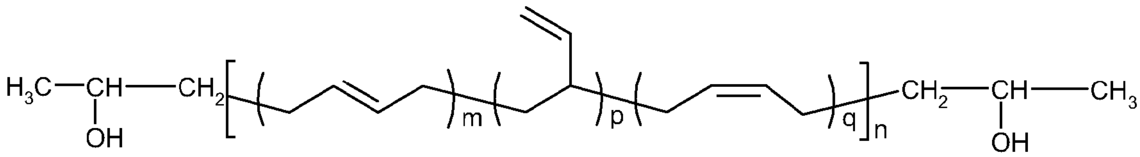

qui sont des copolymères statistiques de 1,4-cis-butadiène et de 1,4-transbutadiène, avec m=0,17, p=0,65 et q=0,18 (fractions molaires), et n est tel que le poids moléculaire moyen en nombre Mn est compris entre 1000 et 12000, notamment entre 2000 et 10000 ($g.mol^{-1}$).

On peut en particulier citer les produits commerciaux KRASOL LBH 2000, 3000, 5000 ou 10000 de Sartomer.

Dans un second mode de réalisation, ladite polyoléfine peut comprendre en outre des unités additionnelles ne comprenant pas de double liaison C=C.

Toutefois, ces unités additionnelles sont présentes en une quantité maximale de 90% molaire, étant donné que la polyoléfine finale doit comprendre au moins 10% molaire d'unités comprenant au moins une double liaison C=C.

Ces unités oléfines additionnelles peuvent notamment être choisies parmi les unités éthylène $-(CH_2-CH_2)_n-$, propylène $-(CH_2-CH_2-CH_2)_n-$, isopropylène - $(CH_2CH(CH_3))_n-$, et/ou butylène de formule :

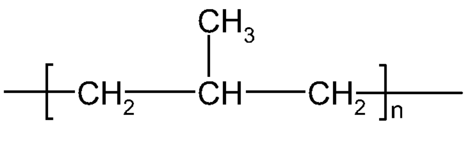

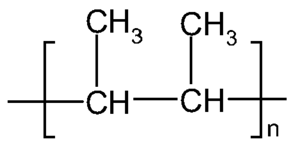

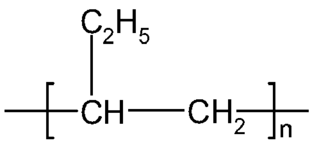

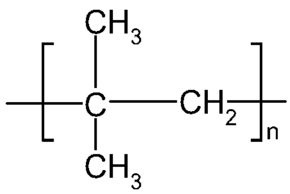

ainsi que leurs mélanges.

Les homopolymères ou copolymères d'oléfines telles que définies ci-dessus peuvent subir, ultérieurement à la polymérisation, une hydrogénation partielle des doubles liaisons résiduelles. Cette hydrogénation ne peut en aucun cas être totale.

En effet, les polyoléfines susceptibles d'être employées pour former les motifs (a2) selon l'invention doivent obligatoirement comprendre au moins 10% en mole d'unités comprenant au moins une double liaison C=C (résiduelle), par rapport à la totalité des unités formant ladite polyoléfine.

Elles comprennent de préférence au moins 20% molaire, notamment au moins 40% molaire, voire au moins 50% molaire, préférentiellement au moins 80% molaire, et tout particulièrement 100% molaire, d'unités comprenant au moins une double liaison C=C, notamment comprenant une seule double liaison C=C.

Cette teneur en unité comportant au moins une double liaison C=C peut notamment être déterminée par les techniques usuelles, notamment par RMN ou par dosage à l'iode.

De préférence, la ou les polyoléfines formant les motifs non ioniques (a2) ont une masse moléculaire en nombre (Mn) comprise entre 400 et 50000, de préférence entre 500 et 30000, mieux entre 1000 et 15000, et encore mieux entre 1500 et 12000.

De préférence, les polyoléfines susceptibles d'être utilisées dans le cadre de l'invention sont :

- des homopolymères tels que le 1,4-polybutadiène et le 1,2-polybutadiene;
- des copolymères de structure :

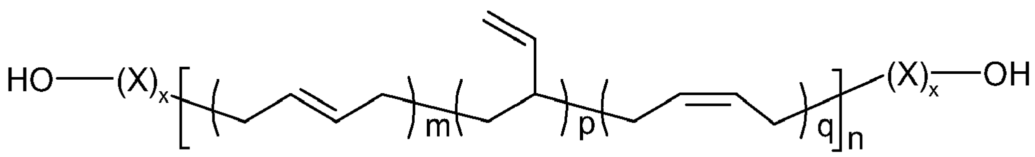

avec

m, n et p étant des fractions molaires comprises entre 0 et 1, et m+n+p=1;

notamment m compris entre 0,1 et 0,8, voire 0,15 et 0,7; p compris entre 0,1 et 0,8, voire 0,15 et 0,7; et q compris entre 0,05 et 0,5, voire 0,1 et 0,4;

n est un entier compris entre 10 et 100, notamment entre 15 et 50;

x = 0 ou 1, et

X = radical carboné divalent, notamment alkylène, linéaire, cyclique ou ramifié, comprenant 1 à 10 atomes de carbone; notamment méthylène, éthylène, propylène ou isopropylène.

Le polyuréthane cationique selon l'invention comprend également au moins un motif (b) résultant d'au moins un composé comportant au moins deux fonctions isocyanate.
Il peut bien évidemment s'agir d'un mélange de plusieurs composés comportant au moins deux fonctions isocyanate.

Les composés comportant au moins deux fonctions isocyanate peuvent être choisis parmi les diisocyanates, ou les mélanges d'un diisocyanate et d'un polyisocyanate comportant plus de deux fonctions isocyanates, ledit polyisocyanate représentant de préférence 0,1 à 40% en poids dudit mélange, notamment 0,5% à 35% en poids, voire 1 à 30% en poids du poids dudit mélange.

Les composés comportant au moins deux fonctions isocyanate peuvent de préférence être choisis parmi les diisocyanates aliphatiques, cycliques conjugués ou non, aromatiques ou non. Ils peuvent notamment être choisis parmi le méthylène-diphényldiisocyanate, le méthylènecyclohexanediisocyanate, l'isophorone diisocyanate, le toluène diisocyanate, le naphtalène diisocyanate, le 1,4-butanediisocyanate et le 1,6-hexane-diisocyanate, et leur mélange; de préférence l'isophorone diisocyanate.

Préférentiellement, le polyuréthanne selon l'invention est constitué essentiellement :

- d'au moins un motif cationique résultant d'amines de formule :

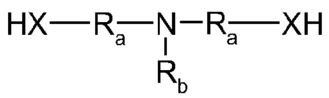
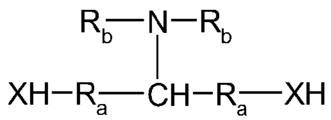
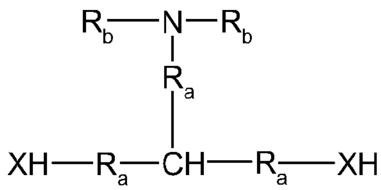

dans lesquelles :

Ra est un groupe divalent alkylène en $C_1$-$C_6$, linéaire ou ramifié, notamment méthylène ou éthylène;

Rb est un groupe alkyle linéaire ou ramifié, en $C_1$-$C_6$, notamment un groupe méthyle, éthyle, n-butyle, isobutyle ou tert-butyle;

etX=O;

- d'au moins un motif non ionique résultant de polyoléfines choisies parmi les homopolymères 1,4-polybutadiène et 1,2-polybutadiene; ou les copolymères de structure :

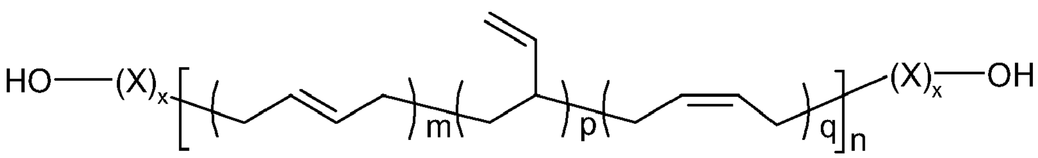

avec
m, n et p étant des fractions molaires comprises entre 0 et 1, et m+n+p=1;
notamment m compris entre 0,1 et 0,8, voire 0,15 et 0,7; p compris entre 0,1 et 0,8, voire 0,15 et 0,7; et q compris entre 0,05 et 0,5, voire 0,1 et 0,4;
n est un entier compris entre 10 et 100, notamment entre 15 et 50;
x = 0 ou 1, et
X = radical carboné divalent, notamment alkylène, linéaire, cyclique ou ramifié, comprenant 1 à 10 atomes de carbone; notamment méthylène, éthylène, propylène ou isopropylène.

- d'au moins un motif résultant de diisocyanates aliphatiques.

Encore plus préférentiellement, les polyuréthannes selon l'invention sont constitués essentiellement

- d'au moins un motif cationique résultant d'amines de formule :

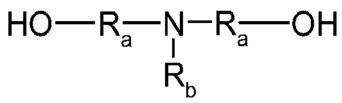

dans laquelle Ra est un groupe divalent alkylène en $C_1$-$C_6$, linéaire ou ramifié, notamment méthylène ou éthylène; et Rb est un groupe alkyle linéaire ou ramifié, en $C_1$-$C_6$, notamment un groupe méthyle, éthyle, n-butyle, isobutyle ou tert-butyle;
et plus particulièrement la N-méthyldiéthanolamine et la N-tert-butyldiéthanolamine;

- d'au moins un motif non ionique résultant de polyoléfines de structure :

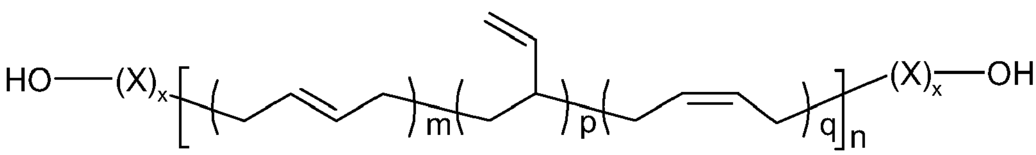

avec
m, n et p étant des fractions molaires comprises entre 0 et 1, et m+n+p=1;
notamment m compris entre 0,1 et 0,8, voire 0,15 et 0,7; p compris entre 0,1 et 0,8, voire 0,15 et 0,7; et q compris entre 0,05 et 0,5, voire 0,1 et 0,4;
n est un entier compris entre 10 et 100, notamment entre 15 et 50;
x = 0 ou 1, et
X = radical carboné divalent, notamment alkylène, linéaire, cyclique ou ramifié, comprenant 1 à 10 atomes de carbone; notamment méthylène, éthylène, propylène ou isopropylène.

- d'au moins un motif résultant de diisocyanates choisis parmi le méthylènecyclohexanediisocyanate, l'isophorone diisocyanate, le 1,4-butanediisocyanate et le 1,6-hexane-diisocyanate, et leur mélange; de préférence l'isophorone diisocyanate.

Le polyuréthanne selon l'invention est constitué essentiellement de motifs (a1), (a2) et (b) tels que définis ci-dessus, ce qui implique qu'il ne comprend pas de motifs additionnels autres que ceux-ci.
Le paramètre physique caractérisant le mieux les propriétés viscoélastiques du polyuréthane cationique selon l'invention est sa recouvrance instantanée en traction Ri.
Le polyuréthane cationique de la présente invention présente de préférence une recouvrance instantanée ($R_i$), mesurée dans les conditions indiquées ci-dessous, avant les exemples, comprise entre 5% et 95%, en particulier comprise entre 20% et 90% et idéalement entre 50 et 85%, notamment entre 55% et 85%.
La viscosité du polyuréthane selon l'invention, mesurée à 10% dans le tétrahydrofuranne (THF), à 25°C, avec un viscosimètre Brookfield, module aiguille, est généralement comprise entre 1 et 1000 cps, de préférence entre 1 et 100 cps, mieux entre 2 et 80 cps. Le polyuréthane est caractérisé sous forme non neutralisée.

Le polyuréthane selon la présente invention présente de préférence au moins deux températures de transition vitreuse (Tg) dont une au moins est inférieure de préférence à 10°C, de préférence inférieure à 0°C et mieux encore inférieure à -10°C, et au moins une autre est de préférence supérieure ou égale à la température ambiante (20°C).

La recouvrance instantanée et par conséquent les propriétés viscoélastiques du polyuréthane selon l'invention dépend de la nature et de la quantité des différents motifs (a1), (a2) et (b).

La fraction de motifs cationiques (a1) doit de préférence être suffisante pour conférer aux polymères leur charge positive responsable de leur bonne affinité pour les substrats kératiniques.
Le ou les motifs non ioniques (a2) doivent de préférence représenter une fraction en poids suffisante pour que les polyuréthanes présentent au moins une température de transition vitreuse inférieure à 10°C et ne forment pas des films cassants.

De préférence, les amines formant les motifs cationiques ou cationisables (a1) représentent de 0,1 à 50%, de préférence de 1 à 30%, mieux de 5 à 20% en poids, du poids total du polyuréthanne final.
De préférence, les polyoléfines formant les motifs non ioniques (a2) représentent de 30 à 99% en poids, de préférence de 50 à 90%, mieux de 60 à 80% en poids, du poids total de polyuréthanne final.
De préférence, les composés comprenant au moins deux fonctions isocyanate, formant les motifs (b) sont présents en une quantité essentiellement stoechiométrique par rapport à la somme des amines tertiaires/quaternaires formant les motifs (a1) et des polyoléfines formant les motifs (a2).
En effet, l'obtention de polyuréthanes ayant des masses molaires importantes suppose un nombre de fonctions isocyanate pratiquement identique au nombre de fonctions à hydrogène labile. L'homme du métier saura choisir un éventuel excès molaire de l'un ou de l'autre type de fonction pour ajuster la masse molaire à la valeur désirée.
Ainsi, de préférence, les composés comprenant au moins deux fonctions isocyanate formant les motifs (b) représentent 1 à 60% en poids, notamment 5 à 50% en poids, mieux 15 à 35% en poids, du poids total du polyuréthanne final.

Afin de former le polyuréthanne selon l'invention, on emploie de préférence :

- 20 à 55%, notamment 25 à 50%, voire de 30 à 47% molaire d'amine(s) tertiaire et/ou quaternaire susceptible(s) de former les motifs (a1);
- 1 à 30%, notamment 2 à 25%, voire de 3 à 20% molaire de polyoléfine(s) susceptible(s) de former les motifs (a2); et
- 30 à 65%, notamment 35 à 60%, voire de 45 à 55% molaire de composé(s) comportant au moins deux fonctions isocyanate susceptible(s) de former les motifs (b).

Préférentiellement, le rapport molaire entre (b) et (a1)+(a2) est proche de 1, notamment compris entre 0,9 et 1,1.

Le polyuréthane cationique ou cationisable à caractère élastique selon l'invention trouve une application toute particulière dans les domaines cosmétique et pharmaceutique. Ainsi, il peut être incorporé dans de nombreuses compositions cosmétiques dont il va améliorer les propriétés cosmétiques, notamment de coiffage.

La quantité de polyuréthane présente dans les compositions dépend bien entendu du type de composition et des propriétés recherchées et peut varier à l'intérieur d'une gamme très large, comprise généralement entre 0,1 et 90% en poids, de préférence entre 1 et 50% en poids, notamment entre 2 et 25% en poids, voire entre 5 et 15% en poids, et mieux entre 6 et 10% en poids, par rapport au poids de la composition cosmétique ou pharmaceutique finale.

Ainsi, lorsque le polyuréthane selon l'invention est destiné à être incorporé dans des compositions capillaires de type laque pour cheveux, sa teneur est de préférence comprise entre 0,1 et 25% en poids, notamment entre 1 et 20% en poids, mieux entre 2 et 15% en poids, voire entre 4 et 8% en poids, par rapport au poids de la composition finale.
Lorsqu'il est destiné à être incorporé dans des compositions de coiffage, il représente de préférence 0,5 à 20% en poids, notamment entre 1 et 15% en poids, mieux entre 2 et 10% en poids, voire entre 5 et 8% en poids, du poids de la composition.
Lorsqu'il est destiné à être incorporé dans des compositions de type shampooing, il représente de préférence 0,1 à 20% en poids, notamment 0,5 à 15% en poids, mieux entre 1 et 10% en poids, voire entre 2 et 5% en poids, du poids de la composition.

Les compositions selon l'invention peuvent se présenter sous toutes les formes galéniques classiquement utilisées pour une application topique et notamment sous forme d'une solution ou suspension aqueuse, alcoolique ou hydroalcoolique, ou d'une solution ou suspension huileuse, ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle de type crème (H/E) ou (E/H), ou d'un gel aqueux ou anhydre, d'un onguent, d'une poudre libre ou compactée à utiliser telle quelle ou à incorporer dans un excipient, ou de toute autre forme cosmétique.

Les compositions cosmétiques ou pharmaceutiques selon l'invention comprennent, outre lesdits polymères, un milieu physiologiquement acceptable, notamment cosmétiquement ou pharmaceutiquement, notamment dermatologiquement, acceptable, c'est-à-dire un milieu compatible avec les tissus cutanés comme la peau du visage ou du corps, et les matières kératiniques telles que les cheveux, les cils, les sourcils et les ongles.

De préférence, le milieu physiologiquement acceptable comprend un milieu solvant ou de dispersion des polymères selon l'invention, qui peut comprendre au moins un composé choisi parmi l'eau, les alcools, les polyols, les esters, les huiles carbonées, les huiles de silicone, les huiles de silicone fluorées, et leurs mélanges.

De préférence, le milieu physiologiquement acceptable des compositions selon l'invention peut comprendre de l'eau ou un mélange d'eau et de solvant(s) organique(s) hydrophile(s) comme les alcools et notamment les monoalcools, linéaires ou ramifiés en $C_1$-$C_6$, comme l'éthanol, le tert-butanol, le n-butanol, l'isopropanol ou le n-propanol, ou le 2-butoxy-éthanol; et les polyols comme la glycérine, la diglycérine, l'éthylène glycol, le propylène glycol, le sorbitol, le pentylène glycol, et les polyéthylène glycols, ou bien encore les éthers de polyols ou de glycols notamment en $C_2$ tel que le monoéthyléther et le monométhyléther de diéthylèneglycol, et les aldéhydes en $C_2$-$C_4$ hydrophiles.

La composition selon l'invention peut comprendre en outre au moins un adjuvant cosmétiquement acceptable classiquement utilisé dans les compositions cosmétiques destinées à être appliquées sur les fibres kératiniques.

On peut citer en particulier, à titre d'adjuvant cosmétiquement acceptable, les gélifiants et/ou épaississants; les polymères, associatifs ou non; les tensioactifs anioniques, non ioniques, cationiques et/ou amphotères; les agents propénétrants, les émulsionnants, les parfums, les conservateurs, les charges, les filtres solaires; les matières colorantes, les protéines, les vitamines, les provitamines; les polymères, fixants ou non fixants, anioniques, non ioniques, cationiques ou amphotères; les agents hydratants, les émollients, les agents adoucissants; les huiles minérales, végétales ou synthétiques; les actifs hydrophiles ou lipophiles comme les céramides et les pseudocéramides; les agents anti-mousse, les agents antiperspirants, les agents anti-radicaux libres, les agents bactéricides et les agents anti-pelliculaires.

L'homme de métier veillera à choisir les éventuels additifs et leur quantité de manière à ce qu'ils ne nuisent pas aux propriétés des compositions de la présente invention.

De préférence, la composition selon l'invention peut se présenter sous la forme d'une lotion, épaissie ou non, d'une crème, épaissie ou non, d'un gel, d'une mousse ou de toute autre forme appropriée. Elle peut éventuellement être conditionnée dans un flacon pompe ou dans un récipient aérosol.

La composition cosmétique selon l'invention peut se présenter sous la forme d'un produit de soin, de nettoyage et/ou de maquillage de la peau du corps ou du visage, des lèvres, des cils, des ongles et des cheveux, d'un produit solaire ou autobronzant, d'un produit d'hygiène corporelle, d'un produit capillaire, notamment de soin, de nettoyage, de coiffage ou de coloration des cheveux.

Elle trouve notamment une application particulièrement intéressante dans le domaine capillaire, notamment pour le maintien de la coiffure ou la mise en forme des cheveux, ou encore le nettoyage des cheveux. Les compositions capillaires sont de préférence des shampooings, des après-shampooings, des gels de coiffage ou de soin, des lotions ou crèmes de soin, des conditionneurs, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Elle peut notamment se présenter sous la forme d'un produit de coloration capillaire; ou sous forme de composition pour permanente, défrisage, ou décoloration, ou encore sous forme de compositions à rincer, à appliquer avant ou après une coloration, une décoloration, une permanente ou un défrisage ou encore entre les deux étapes d'une permanente ou d'un défrisage.

La composition selon l'invention peut également se présenter sous la forme d'une composition de soin, notamment hydratant, pour la peau, les lèvres et/ou les phanères, ou sous forme d'une composition de nettoyage de la peau, par exemple un produit démaquillant ou un gel pour le bain ou la douche.

Elle peut aussi se présenter sous forme d'un produit de soin, non coloré, destiné à traiter la peau et notamment à l'hydrater, la lisser, la dépigmenter, la nourrir, la protéger des rayons solaires, ou lui conférer un traitement spécifique.

Elle peut également se présenter sous forme d'une composition d'hygiène corporelle notamment sous forme de produit déodorant, anti-transpirant, ou encore sous forme d'une composition dépilatoire.

Elle peut encore se présenter sous la forme d'un produit de maquillage, en particulier coloré, de la peau du corps ou du visage, ou des cheveux, en particulier un fond de teint, présentant éventuellement des propriétés de soin, un blush, un fard à joues ou à paupières, un produit anti-cerne, un eye-liner; un produit de maquillage des lèvres comme un rouge à lèvres, présentant éventuellement des propriétés de soin, un brillant à lèvres, les crayons à lèvres; un produit de maquillage des phanères comme les ongles, les cils en particulier sous forme d'un mascara pain, les sourcils et les cheveux; un produit de tatouage temporaire de la peau du corps.

Avantageusement, la composition selon l'invention est une composition capillaire de coiffage des cheveux, et peut se présenter sous forme de gel, de mousse ou de spray.

L'invention a aussi pour objet un procédé de traitement cosmétique, notamment de maquillage, de soin, de nettoyage, de coloration, de mise en forme des matières kératiniques, notamment de la peau du corps ou du visage, des ongles, des cheveux, des poils et/ou des cils, comprenant l'application sur lesdites matières d'une composition cosmétique telle que définie précédemment.

De préférence, il s'agit d'un procédé de traitement cosmétique pour la mise en forme et/ou le maintien des cheveux,

comprenant l'application d'une composition selon l'invention sur lesdits cheveux; éventuellement suivie d'une étape de rinçage.

L'invention est illustrée plus en détail dans les exemples suivants.

Détermination des masses molaires

On détermine les masses molaires moyennes en poids (Mw) et en nombre (Mn) par chromatographie liquide par perméation de gel ou GPC (solvant THF, courbe d'étalonnage établie avec des étalons de polystyrène linéaire, détecteur réfractométrique).

L'indice de dispersé est calculé de la manière suivante : Ip = Mw/Mn

La GPC est réalisée avec des colonnes STYRAGEL HR4 /7,8 x 300 mm, vendues par Waters WAT044225.

La détection est effectuée avec un réfractomètre WATERS 410.

L'éluant est le THF (tétrahydrofurane), à un débit de 1 ml/minute.

Le volume injecté est de 50 microlitre, à 25°C.

L'étalonnage est effectué à l'aide de standards de polystyrène.

Détermination de la recouvrance instantanée Ri

Le paramètre physique caractérisant le mieux les propriétés viscoélastiques du polyuréthane selon l'invention est sa recouvrance instantanée en traction.

Cette recouvrance est déterminée par essai de fluage en traction consistant à étirer rapidement une éprouvette jusqu'à un taux d'allongement prédéterminé, puis à relâcher la contrainte et à mesurer la longueur de l'éprouvette.

L'essai de fluage utilisé pour la caractérisation du polyuréthane selon l'invention se déroule de la manière suivante :

On utilise, comme éprouvette, un film du polyuréthane ayant une épaisseur de 500 $\pm$ 50 mm, découpé en bandes de 80 mm x 15 mm. Ce film de copolymère est obtenu par séchage, à une température de 22 $\pm$ 2°C et à une humidité relative de 50 $\pm$ 5%, d'une solution ou dispersion à 3% en poids dudit polyuréthane dans de l'eau et/ou de l'éthanol.

Chaque bande est fixée entre deux mors, distants de 50 $\pm$ 1 mm l'un de l'autre, et est étirée à une vitesse de 20 mm/minute (dans les conditions de température et d'humidité relative ci-dessus) jusqu'à un allongement de 50% ($\varepsilon_{max}$), c'est-à-dire jusqu'à 1,5 fois sa longueur initiale. On relâche alors la contrainte en imposant une vitesse de retour égale à la vitesse de traction, soit 20 mm/minute, et on mesure l'allongement de l'éprouvette (exprimé en % par rapport à la longueur initiale) immédiatement après retour à charge nulle ($\varepsilon_i$).

La recouvrance instantanée ($R_i$) est calculée à l'aide de la formule suivante :

$$R_i (\%) = ((\varepsilon_{max} - \varepsilon_i)/ \varepsilon_{max}) \times 100$$

Quantification de l'hydrophobie du polymère

L'hydrophobie du polymère est mesurée sur des films par la détermination des angles de contact ou angle de goutte.

Lorsqu'une goutte de liquide est déposée sur une surface solide plane, l'angle entre la tangente à la goutte au point de contact et la surface solide est appelé angle de contact (θ). Il rend compte de l'aptitude d'un liquide à s'étaler sur une surface et dépend des interactions entre le liquide et le solide.

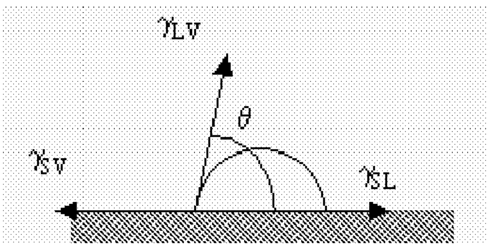


La mesure de cet angle nous donne plusieurs types d'information :

- si on utilise l'eau comme liquide de mesure d'angle de contact, on peut déduire le caractère hydrophobe (faible énergie de surface) ou hydrophile (grande énergie de surface) de la surface. Ainsi plus l'angle de contact est élevé

plus la surface est hydrophobe.

- la mesure de l'hystérésis entre l'angle à l'avancée de la goutte et au retrait de la goutte renseigne sur la non homogénéité physique (rugosité) ou chimique de la surface.

Exemple 1

A/

Dans un réacteur de 1 litre, on introduit :

- 69,7g (12,9% mol) de Polybd R45 HTLO de Sartomer, qui est une résine de polybutadiène, à terminaisons hydroxyle
- 8,15g (35,5% mol) de N-méthyldiéthanolamine (NMDEA),
- 0,025g de dibutyldilaurate d'étain (DBTL) et
- 100g de méthyléthylcétone (MEC).

  On homogénéise le milieu sous agitation et en chauffant à 70°C. Lorsque le milieu est homogène, on introduit 22,1g (51,6% mol) d'isophorone diisocyanate (IPDI) et on chauffe au reflux pendant 8 heures.

  Après 8 heures, une analyse par infrarouge du milieu réactionnel montre une faible bande de NCO résiduel à 2250 $cm^{-1}$. On rajoute 10 g d'éthanol et 130 g de méthyléthylcétone, puis on laisse revenir à température ambiante.

  On obtient une solution du polymère, ayant un extrait sec final de 28%.

B/

Dans un réacteur de 1 litre, on introduit 177 g de solution du polymère ci-dessus à 28% dans la MEC, puis on dilue avec 105 g de THF (tétrahydrofurane). On chauffe à 70°C puis on neutralise 90% molaire des fonctions amines en introduisant 30,8 g d'HCl 1N. On ajoute ensuite 250 g d'eau puis on distille sous vide les solvants organiques pour obtenir un gel aqueux, opaque, d'extrait sec 15,3%.

Exemple 2

A/

Dans un réacteur de 1 litre, on introduit :

- 70 g (11,4% mol) de Krasol LBH-P 3000, qui est un polybutadiène à terminaisons primaires hydroxyle, de Mn = 3200,
- 8,8 g (38,6% mol) de N-méthyldiéthanolamine (NMDEA),
- 0,025g de dibutyldilaurate d'étain (DBTL) et
- 100g de méthyléthylcétone (MEC).

  On homogénéise le milieu sous agitation et en chauffant à 70°C. Lorsque le milieu est homogène, on introduit 21,2 g (49,9% mol) d'isophorone diisocyanate et on chauffe au reflux pendant 8 heures.

  Après 8h, une analyse par infrarouge du milieu réactionnel ne montre plus de trace de NCO résiduel à 2250 $cm^{-1}$.

  On rajoute 10 g d'éthanol et 150 g de MEC puis on laisse revenir à température ambiante.

  On obtient une solution du polymère, ayant un extrait sec final de 28%.

  B/

  Dans un réacteur de 1 litre, on introduit 180 g de solution du polymère ci-dessus à 28% dans la MEC. On chauffe à 70°C puis on neutralise 90% molaire des fonctions amines en introduisant 33,2 g d'HCl 1 N. On ajoute ensuite 87 g d'eau puis 70 g d'éthanol pour homogénéiser le milieu. On complète la dilution avec 370g d'eau puis on homogénéise en rajoutant 110g de THF. On distille sous vide les solvants organiques pour obtenir un gel aqueux, opaque, d'extrait sec 10,9%.

  La recouvrance instantanée ($R_i$) est Ri=75,5 %

Exemple 3

A/

Dans un réacteur de 1 litre, on introduit :

- 70,9 g (7,9% mol) de Krasol LBH 5000, qui est un polybutadiène à terminaisons secondaires hydroxyle, de Mn = 3000,
- 9,1g (42,3% mol) de N-méthyldiéthanolamine (NMDEA),
- 0,025g de dibutyldilaurate d'étain (DBTL) et
- 100g de méthyléthylcétone (MEC).

  On homogénéise le milieu sous agitation et en chauffant à 70°C. Lorsque le milieu est homogène, on introduit 20 g (49,8% mol) d'isophorone diisocyanate et on chauffe au reflux pendant 8 heures.

Après 8h, une analyse par infrarouge du milieu réactionnel ne montre plus de trace de NCO résiduel à 2250 $cm^{-1}$. On rajoute 10 g d'éthanol et 150g de MEC puis on laisse revenir à température ambiante.
On obtient le polymère en solution, avec un extrait sec final de 28%.

B/
Dans un réacteur de 1 litre, on introduit 175g de solution du polymère ci-dessus à 28% dans la MEC. On dilue avec 100 g de THF puis on chauffe à 70°C. On neutralise 90% molaire des fonctions amines en introduisant 34,4g d'HCl 1N. On ajoute ensuite 250 g d'eau puis 150 g d'éthanol pour homogénéiser le milieu. On distille sous vide les solvants organiques pour obtenir une dispersion aqueuse blanche opaque, d'extrait sec 17,6% et de pH = 4,1.

<u>Exemple 4</u>

A/
Dans un réacteur de 1 litre, on introduit :

- 70,5g (4% mol) de Krasol LBH 10000, qui est un polybutadiène à terminaisons secondaires hydroxyle, de Mn = environ 10 000
- 9,8g (46,2% mol) de N-méthyldiéthanolamine (NMDEA),
- 0,025g de dibutyldilaurate d'étain (DBTL) et
- 100g de méthyléthylcétone (MEC).
  On homogénéise le milieu sous agitation et en chauffant à 70°C. Lorsque le milieu est homogène, on introduit 19,7g (49,8% mol) d'isophorone diisocyanate et on chauffe au reflux pendant 8 heures.
  Après 8h, une analyse par infrarouge du milieu réactionnel ne montre plus de trace de NCO résiduel à 2250 $cm^{-1}$. On rajoute 10 g d'éthanol et 150g de MEC puis on laisse revenir à température ambiante.
  On obtient le polymère en solution, avec un extrait sec final de 29%.

B/
Dans un réacteur de 1 litre, on introduit 171 g de solution du polymère ci-dessus à 29% dans la MEC. On rajoute 100 g de THF et on chauffe à 70°C. On neutralise 90% molaire des fonctions amines en introduisant 37g d'HCl 1 N. On ajoute ensuite 250 g d'eau. On distille sous vide les solvants organiques pour obtenir une dispersion aqueuse blanche opaque, d'extrait sec 17,6%.

<u>Exemple 5</u>

A/
Dans un réacteur de 1 litre, on introduit :

- 67,9g (16% mol) de Krasol LBH-P 2000, qui est un polybutadiène à terminaisons primaires hydroxyle, de Mn = 2100,
- 8,7g (34,4% mol) de N-méthyldiéthanolamine (NMDEA),
- 0,025g de dibutyldilaurate d'étain (DBTL) et
- 100g de méthyléthylcétone (MEC).
  On homogénéise le milieu sous agitation et en chauffant à 70°C. Lorsque le milieu est homogène, on introduit 23,4g (49,6% mol) d'isophorone diisocyanate et on chauffe au reflux pendant 8 heures.
  Après 8h, une analyse par infrarouge du milieu réactionnel montre l'absence de bande NCO à 2250 $cm^{-1}$. On rajoute 10 g d'éthanol et 150g de THF puis on laisse revenir le milieu réactionnel à température ambiante.
  On obtient le polymère en solution, avec un extrait sec final de 25,5%.

B/
Dans un réacteur de 1 litre, on introduit 196 g de solution du polymère ci-dessus à 25,5% dans le mélange MEC/THF. On chauffe à 70°C puis on neutralise 90% molaire des fonctions amines en introduisant 32,9 g d'HCl 1N. On ajoute ensuite 300 g d'eau puis on évapore sous vide le milieu réactionnel pour obtenir une dispersion opalescente d'extrait sec 14,4% et de pH=4,5.
La recouvrance instantanée ($R_i$) est Ri=72%
Masse : Mp=48900g/mol; Mn=31100g/mol; Mw=56800g/mol Ip=1,8
C/
Dans un réacteur de 1 litre, on introduit 38 g de polymère sec, obtenu par évaporation des solvants et séchage de la solution préparée à l'étape A, que l'on solubilise dans 38 g de MEC en chauffant à 70°C. On neutralise 40% molaire des fonctions amines en ajoutant 11g d'HCl1N. On rajoute alors 77g d'eau et on évapore sous vide la MEC. On obtient

alors un latex (dispersion) opalescent fluide.

Exemple 6

La synthèse est identique à celle de l'exemple 5 sauf que 0,01 % de catalyseur est utilisé à la place de 0,025%.

A/

Dans un réacteur de 1 litre, on introduit :

- 203,7g de Krasol LBH-P 2000,
- 26,1g de N-Méthyldiéthanol amine (NMDEA),
- 0,03g de dibutyldilaurate d'étain (DBTL) et
- 300g de méthyléthylcétone (MEC).
  On homogénéise le milieu sous agitation et en chauffant à 70°C. Lorsque le milieu est homogène, on introduit 70,2g d'isophorone diisocyanate et on chauffe au reflux pendant 8 heures.
  Après 8h, une analyse par infrarouge du milieu réactionnel montre l'absence de bande NCO à 2250 $cm^{-1}$. On rajoute 30g d'éthanol puis on laisse revenir le milieu réactionnel à température ambiante.
  On obtient le polymère en solution, avec un extrait sec final de 53,5%.
  Masse : Mp=58300g/mol; Mn=37500g/mol; Mw=70700g/mol Ip=1,9

B/

Dans un réacteur de 1 litre, on introduit 187 g de solution du polymère à 53,5% préparée à l'étape A; on ajoute 13 g de MEC pour obtenir une solution d'extrait sec de 50%, puis on chauffe à 70°C. On neutralise 40% molaire des fonctions amines en ajoutant 29,2 g d'HCl1N. On rajoute ensuite 204 g d'eau et on évapore sous vide la MEC. On obtient alors une dispersion opalescente d'extrait sec 30,1 %.

La recouvrance instantanée ($R_i$) est Ri=72%

C/

Dans un réacteur de 1 litre, on introduit 58,9 g de solution du polymère à 53,5% préparée à l'étape A ci-dessus; on ajoute 8 g de MEC pour obtenir une solution ayant un extrait sec de 50%. On chauffe à 70°C puis on neutralise 90% molaire des fonctions amines en introduisant 38,7 g d'HCl 1 N. On ajoute ensuite 295 g d'eau puis on évapore sous vide la MEC. On obtient alors un latex opalescent fluide d'extrait sec 14,8%.

La recouvrance instantanée ($R_i$) est Ri=73%

D/

Dans un tricol de 500 ml, on introduit 20 g du polymère obtenu à l'étape A ci-dessus, que l'on dilue à 20% dans la MEC. On rajoute 207,3 g de iodométhane (Mel) et on laisse agiter à température ambiante pendant 24 heures. Après 24 heures, le milieu a gélifié et pris une teinte jaune.

On distille 200 g du mélange MEC/Mel en chauffant à 100°C. On rajoute ensuite 200 g de MEC et on redistille 200 g de solvant pour éliminer les traces de iodométhane résiduel.

On ajoute ensuite 162 g d'eau sous agitation et on récupère un milieu biphasique que l'on évapore sous vide.

On obtient un latex blanc d'extrait sec 13,1% et de pH=5,5

La recouvrance instantanée ($R_i$) est Ri=75%

Masse : Mp=58300g/mol; Mn=37500g/mol; Mw=70700g/mol Ip=1,9

Exemple comparatif 7

On prépare un polymère comparatif, selon WO02/32978.

A/

Dans un réacteur de 1 litre, on introduit :

- 70 g (12,5% mol) de PTMO (polytétraméthylène oxyde) de Mn =2900,
- 8,6 g (37,5% mol) de N-méthyldiéthanolamine (NMDEA),
- 0,025g de dibutyldilaurate d'étain (DBTL) et
- 100g de méthyléthylcétone (MEC).
  On homogénéise le milieu sous agitation et en chauffant à 70°C. Lorsque le milieu est homogène, on introduit 21,4 g (50% mol) d'isophorone diisocyanate et on chauffe au reflux pendant 8 heures.
  Après 8h, une analyse par infrarouge du milieu réactionnel montre l'absence de bande NCO à 2250 $cm^{-1}$. On rajoute 10 g d'éthanol puis on laisse revenir le milieu réactionnel à température ambiante.
  On obtient le polymère en solution, avec un extrait sec final de 50%.

B/
Dans un réacteur de 1 litre, on introduit 200 g de solution du polymère ci-dessus à 50% d'extrait sec. On chauffe à 70°C puis on neutralise 90% molaire des fonctions amines en introduisant 64,9 g d'HCl 1 N. On ajoute ensuite 400 g d'eau puis on évapore sous vide le milieu réactionnel pour obtenir une dispersion opalescente d'extrait sec 20%.
C/
On compare les trois polymères suivants :

| | Prépolymère | NMDEA | IPDI | Neutralisation |
|---|---|---|---|---|
| Polymère comparatif 7B | PTMO 2900 70% | 8,6% | 21,4% | 90% des motifs amines |
| Polymère Invention 5B | Krasol LBH P2000 67,9% | 8,7% | 23,4% | 90% des motifs amines |
| Polymère Invention 5C | Krasol LBH P2000 67,9% | 8,7% | 23,4% | 40% des motifs amines |

Réalisation des films :
Les dispersions de polymère des exemples 5B, 5C et 7B sont diluées à 13% par de l'eau HPLC. On dépose ensuite 2 ml de dispersion sur des plaques en verre de 1 cm x 5cm. On laisse sécher les films à température et humidité ambiante (20°C) pendant 24 heures.
Mesure des angles de contact :
Marque de l'appareil : GBX
Nom de l'appareil : DGD Fast 160 Contact Angle Meter
Paramètres : Méthode : Manuel 2 ; Photos à 100ms ; Volume de la goutte 3.5μl ; Déplacement vertical : 95
Liquide utilisé : eau
Les mesures sont réalisées sur les angles gauche et droite de la goutte; plusieurs mesures sont réalisées de façon à obtenir une valeur moyenne.
La valeur des angles de contact mesurés est donnée dans le tableau ci-dessous.

| | | Angle gauche en ° | Angle droite en ° | θ moyenne |
|---|---|---|---|---|
| Exemple comparatif 7B | Mesure 1 | 81.9 | 81.9 | 79.6 |
| | Mesure 2 | 76.6 | 76.6 | |
| | Mesure 3 | 80.4 | 79.9 | |
| Exemple 5B | Mesure 1 | 92.8 | 92.8 | 90.1 |
| | Mesure 2 | 87.0 | 87.0 | |
| | Mesure 3 | 90.5 | 90.5 | |
| Exemple 5C | Mesure 1 | 100.6 | 100 | 99.8 |
| | Mesure 2 | 97.7 | 99.1 | |

Le polymère comparatif est nettement plus hydrophile que les polymères selon l'invention. L'incorporation d'un segment polybutadiène permet donc de rendre le matériau plus hydrophobe et plus résistant à l'humidité.
Par ailleurs, on constate que le dépôt effectué sur une mèche de cheveux ("méché") sur laquelle on a appliqué le polymère comparatif 7B (en lotion, imprégnation, séchage au casque) est moins résistant à l'eau que celui obtenu avec les polymères 5B ou 5C.
Le polymère comparatif 7B forme un mèche collant, alors que les polymères 5B et 5C selon l'invention donnent des méchés non collants.

<u>Exemple 8</u>

On prépare une composition capillaire de type laque comprenant :

- Polymère de l'exemple 1 6% MA (matière active)
- eau déminéralisée qsp 100%
- Diméthyléther : 35g de gaz pour 65g de la dispersion de polymère à 6%

Exemple 9

On prépare une composition capillaire de type mousse de coiffage comprenant :

- Polymère de l'exemple 1 6% MA (matière active)
- eau déminéralisée qsp 100%
- Mélange isobutane / propane / butane : 5g de gaz pour 95g de la dispersion de polymère à 6%

## Revendications

**1.** Polyuréthane constitué essentiellement :

(a1) d'au moins un motif cationique ou cationisable dérivé d'au moins une amine tertiaire ou quaternaire présentant au moins deux fonctions réactives à hydrogène labile,
(a2) d'au moins un motif non ionique dérivé d'au moins une polyoléfine présentant au moins deux fonctions réactives à hydrogène labile, ladite polyoléfine comprenant au moins 10% en mole d'unités comprenant au moins une double liaison C=C, par rapport à la totalité des unités formant ladite polyoléfine;
(b) d'au moins un motif dérivé d'un composé comportant au moins deux fonctions isocyanate.

**2.** Polyuréthane selon la revendication 1, dans lequel les amines formant les motifs (a1) sont choisies parmi les composés de formule :

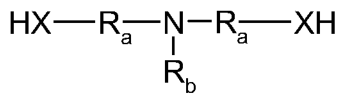
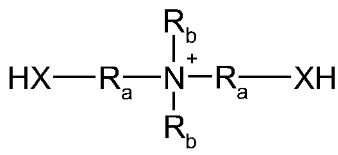
A-

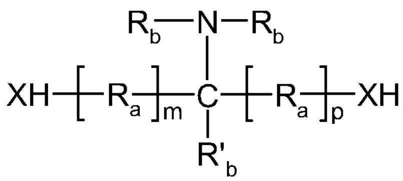
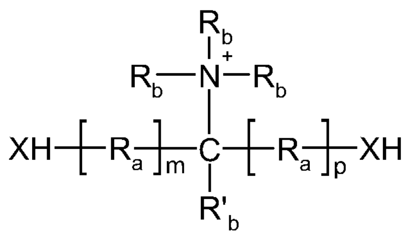
A-

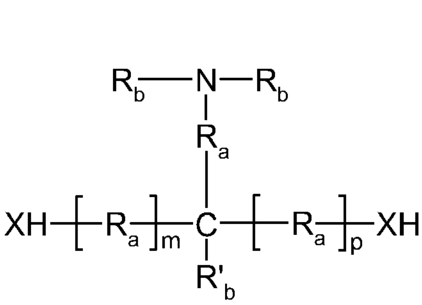
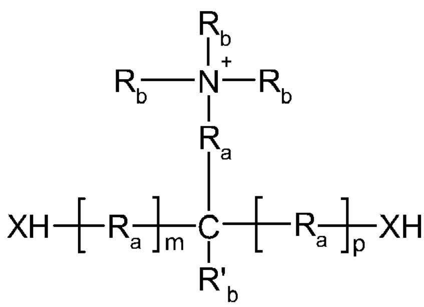
A-

dans lesquelles :

- chaque $R_a$, indépendamment les uns des autres, représente un groupe divalent alkylène en $C_1$-$C_6$, linéaire ou ramifié, ou bien cycloalkylène en $C_3$-$C_6$ ou arylène,

ou leurs mélanges; ces groupes pouvant être substitués par un ou plusieurs atomes d'halogène et/ou comporter

un ou plusieurs hétéroatomes choisis parmi O, N, P et S,

- chaque $R_b$ représente indépendamment les uns des autres, un groupe alkyle linéaire ou ramifié, en $C_1$-$C_6$, ou bien cycloalkyle en $C_3$-$C_6$ ou encore aryle, ou leurs mélanges; ces groupes pouvant être substitués par un ou plusieurs atomes d'halogène et/ou comporter un ou plusieurs hétéroatomes choisis parmi O, N, P et S,
- chaque R'b représente H ou un groupe alkyle linéaire ou ramifié, en $C_1$-$C_6$, ou bien cycloalkyle en $C_3$-$C_6$ ou encore aryle, ou leurs mélanges; ces groupes pouvant être substitués par un ou plusieurs atomes d'halogène et/ou comporter un ou plusieurs hétéroatomes choisis parmi O, N, P et S,
- m et p sont, indépendamment l'un de l'autre, égaux à 0 ou 1; de préférence m= 1 etp=1;
- chaque X représente, indépendamment les uns des autres, un atome d'oxygène ou de soufre, ou un groupe NH ou $NR_c$, où $R_c$ représente un groupe alkyle en $C_1$-$C_6$, et
- $A^-$ représente un contre-ion physiologiquement acceptable, et notamment un halogénure tel que chlorure ou bromure.

**3.** Polyuréthane selon l'une des revendications précédentes, dans lequel les amines sont :

- de formule :

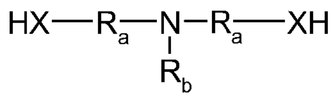

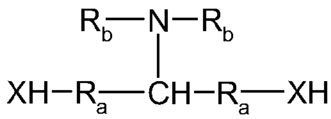

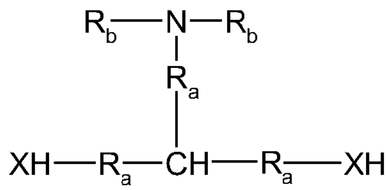

et/ou
- Ra est un groupe divalent alkylène en $C_1$-$C_6$, linéaire ou ramifié, notamment méthylène ou éthylène; et/ou
- Rb est un groupe alkyle linéaire ou ramifié, en $C_1$-$C_6$, notamment un groupe méthyle, éthyle, n-butyle, isobutyle ou tert-butyle; et/ou
- X=O.

**4.** Polyuréthane selon l'une des revendications précédentes, dans lequel les amines sont de formule :

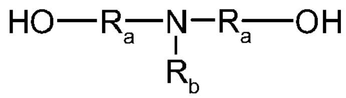

dans laquelle Ra est un groupe divalent alkylène en $C_1$-$C_6$, linéaire ou ramifié, notamment méthylène ou éthylène; et Rb est un groupe alkyle linéaire ou ramifié, en $C_1$-$C_6$, notamment un groupe méthyle, éthyle, n-butyle, isobutyle ou tert-butyle.

**5.** Polyuréthane selon l'une des revendications précédentes, dans lequel les amines sont choisies parmi la N-méthyldiéthanolamine et la N-tert-butyldiéthanolamine.

**6.** Polyuréthane selon l'une des revendications précédentes, dans lequel la polyoléfine présentant au moins deux fonctions réactives à hydrogène labile, formant les motifs non ioniques (a2) est un homopolymère d'oléfines.

**7.** Polyuréthane selon la revendication 6, dans lequel l'homopolymère est choisi parmi les homopolymères de 1,2-butadiène, de 1,4-butadiène ou d'isoprène, et notamment les 1,4-polybutadiène, sous leurs formes cis et trans; les 1,2-polybutadiene, les poly(cis-1,4-isoprene) et les poly(trans-1,4-isoprene).

**8.** Polyuréthane selon l'une des revendications 1 à 5, dans lequel la polyoléfine présentant au moins deux fonctions réactives à hydrogène labile, formant les motifs non ioniques (a2) est un copolymère d'oléfines exclusivement constitué d'unités comprenant au moins une double liaison C=C.

**9.** Polyuréthane selon la revendication 8, dans lequel la polyoléfine est choisie parmi les copolymères, notamment

statistiques, comprenant des unités 1,2-butadiène et/ou des unités 1,4-butadiène dans ses formes cis et/ou trans, et/ou des unités isoprène, notamment cis-1,4-isoprène et trans-1,4-isoprène, en mélange.

**10.** Polyuréthane selon l'une des revendications 8 à 9, dans lequel la polyoléfine est un copolymère statistique (1,2-butadiène/1,4-butadiène).

**11.** Polyuréthane selon l'une des revendications 8 à 9, dans lequel la polyoléfine est statistique et répond à la structure suivante:

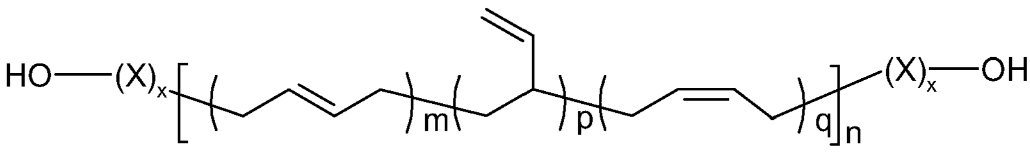

dans laquelle :
m, n et p étant des fractions molaires comprises entre 0 et 1, et m+n+p=1;
notamment m compris entre 0,1 et 0,8, voire 0,15 et 0,7; p compris entre 0,1 et 0,8, voire 0,15 et 0,7; et q compris entre 0,05 et 0,5, voire 0,1 et 0,4;
n est un entier compris entre 10 et 100, notamment entre 15 et 50;
x = 0 ou 1, et
X = radical carboné divalent, notamment alkylène, linéaire, cyclique ou ramifié, comprenant 1 à 10 atomes de carbone; notamment méthylène, éthylène, propylène ou isopropylène.

**12.** Polyuréthane selon la revendication 11, dans lequel la polyoléfine est choisie parmi :

- les polybutadiènes à terminaisons hydroxy, tels que les polymères de structure :

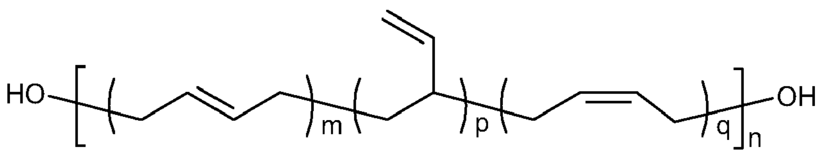

avec m = 0,6, p = q= 0,2 et n= 25;
- les polybutadiènes à terminaisons primaires hydroxy, tels que les polymères de structure :

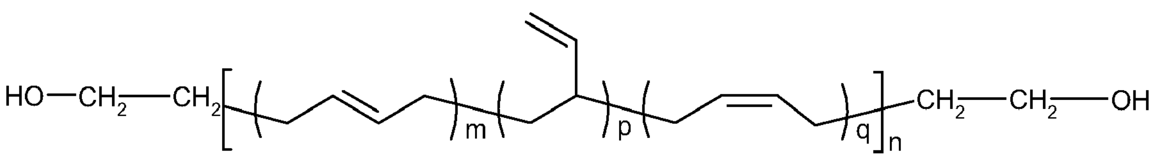

avec m=0,17, p=0,65 et q=0,18, et n est tel que le poids moléculaire moyen en nombre Mn est compris entre 1000 et 10000, notamment entre 2000 et 6000;
- les polybutadiènes à terminaisons secondaires hydroxy, tels que les polymères de structure suivante :

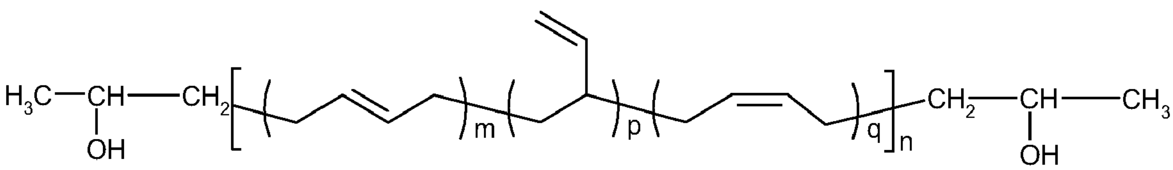

avec m=0,17, p=0,65 et q=0,18 et n est tel que le poids moléculaire moyen en nombre Mn est compris entre 1000 et 12000, notamment entre 2000 et 10000.

**13.** Polyuréthane selon l'une des revendications 1 à 5, dans lequel la polyoléfine présentant au moins deux fonctions réactives à hydrogène labile, formant les motifs non ioniques (a2) est un copolymère comprenant en outre des unités additionnelles ne comprenant pas de double liaison C=C, présentes en une quantité maximale de 90% molaire.

**14.** Polyuréthane selon la revendication 13, dans lequel les unités additionnelles sont choisies parmi les unités éthylène $-(CH_2-CH_2)_n-$, propylène $-(CH_2-CH_2-CH_2)_n-$ , isopropylène $-(CH_2CH(CH_3))_n-$, et/ou butylène de formule :

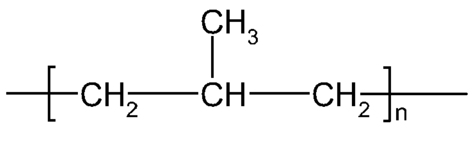

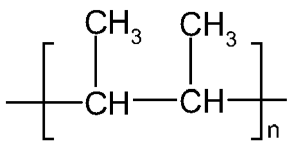

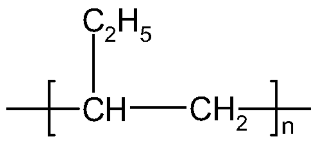

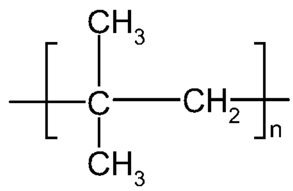

ainsi que leurs mélanges.

**15.** Polyuréthane selon l'une des revendications précédentes, dans lequel les polyoléfines susceptibles d'être employées pour former les motifs (a2) comprennent au moins 20% en mole, notamment au moins 40% molaire, voire au moins 50% molaire, préférentiellement au moins 80% molaire, et tout particulièrement 100% molaire, d'unités comprenant au moins une double liaison C=C, par rapport à la totalité des unités formant ladite polyoléfine.

**16.** Polyuréthane selon l'une des revendications précédentes, dans lequel les polyoléfines susceptibles d'être utilisées sont choisies parmi:

- des homopolymères tels que le 1,4-polybutadiène et le 1,2-polybutadiene;
- des copolymères de structure :

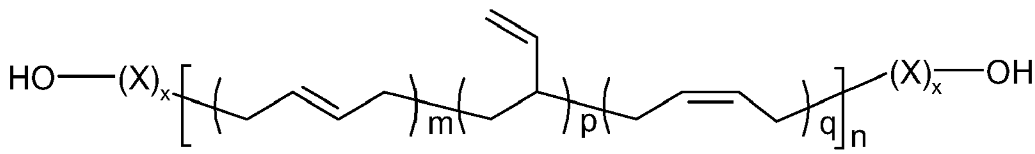

avec
m, n et p étant des fractions molaires comprises entre 0 et 1, et m+n+p=1;
notamment m compris entre 0,1 et 0,8, voire 0,15 et 0,7; p compris entre 0,1 et 0,8, voire 0,15 et 0,7; et q compris entre 0,05 et 0,5, voire 0,1 et 0,4;
n est un entier compris entre 10 et 100, notamment entre 15 et 50;
x = 0 ou 1, et
X = radical carboné divalent, notamment alkylène, linéaire, cyclique ou ramifié, comprenant 1 à 10 atomes de carbone; notamment méthylène, éthylène, propylène ou isopropylène.

**17.** Polyuréthane selon l'une des revendications précédentes, dans lequel la ou les polyoléfines formant les motifs non ioniques (a2) ont une masse moléculaire en nombre (Mn) comprise entre 400 et 50000, de préférence entre 500 et 30000, mieux entre 1000 et 15000, encore mieux entre 1500 et 12000.

**18.** Polyuréthane selon l'une des revendications précédentes, dans lequel le composé comportant au moins deux fonctions isocyanate est choisi parmi les diisocyanates, ou les mélanges d'un diisocyanate et d'un polyisocyanate comportant plus de deux fonctions isocyanates, ledit polyisocyanate représentant de préférence 0,1 à 40% en poids dudit mélange, notamment 0,5% à 35% en poids, voire 1 à 30% en poids du poids dudit mélange.

**19.** Polyuréthane selon l'une des revendications précédentes, dans lequel le composé comportant au moins deux fonctions isocyanate est choisi parmi les diisocyanates aliphatiques, cycliques conjugués ou non, aromatiques ou non.

**20.** Polyuréthane selon l'une des revendications précédentes, dans lequel le composé comportant au moins deux fonctions isocyanate est choisi parmi le méthylènediphényldiisocyanate, le méthylènecyclohexanediisocyanate, l'isophorone diisocyanate, le toluène diisocyanate, le naphtalène diisocyanate, le 1,4-butanediisocyanate et le 1,6-hexane-diisocyanate, et leur mélange; de préférence l'isophorone diisocyanate.

**21.** Polyuréthane selon l'une des revendications précédentes, constitué essentiellement :

- d'au moins un motif cationique résultant d'amines de formule :

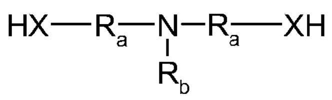

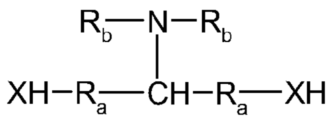

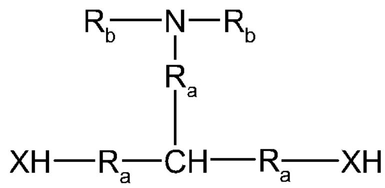

dans lesquelles :

Ra est un groupe divalent alkylène en $C_1$-$C_6$, linéaire ou ramifié, notamment méthylène ou éthylène;
Rb est un groupe alkyle linéaire ou ramifié, en $C_1$-$C_6$, notamment un groupe méthyle, éthyle, n-butyle, isobutyle ou tert-butyle;
et X = O;

- d'au moins un motif non ionique résultant de polyoléfines choisies parmi les homopolymères 1,4-polybutadiène et 1,2-polybutadiene; ou les copolymères de structure :

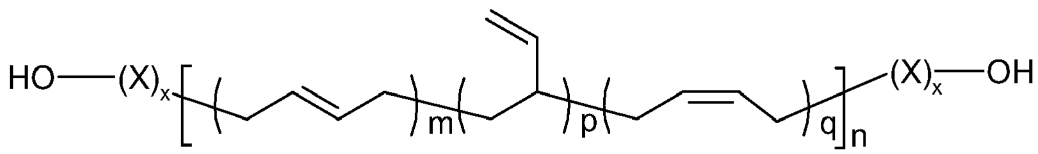

avec
m, n et p étant des fractions molaires comprises entre 0 et 1, et m+n+p=1;
notamment m compris entre 0,1 et 0,8, voire 0,15 et 0,7; p compris entre 0,1 et 0,8, voire 0,15 et 0,7; et q compris entre 0,05 et 0,5, voire 0,1 et 0,4;
n est un entier compris entre 10 et 100, notamment entre 15 et 50;
x = 0 ou 1, et
X = radical carboné divalent, notamment alkylène, linéaire, cyclique ou ramifié, comprenant 1 à 10 atomes de carbone; notamment méthylène, éthylène, propylène ou isopropylène.
- d'au moins un motif résultant de diisocyanates aliphatiques.

**22.** Polyuréthane selon l'une des revendications précédentes, constitué essentiellement :

- d'au moins un motif cationique résultant d'amines de formule :

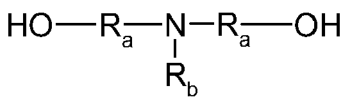

dans laquelle Ra est un groupe divalent alkylène en $C_1$-$C_6$, linéaire ou ramifié, notamment méthylène ou éthylène; et Rb est un groupe alkyle linéaire ou ramifié, en $C_1$-$C_6$, notamment un groupe méthyle, éthyle, n-butyle, isobutyle ou tert-butyle; et plus particulièrement la N-méthyldiéthanolamine et la N-tert-butyldiéthanolamine;

- d'au moins un motif non ionique résultant de polyoléfines de structure :

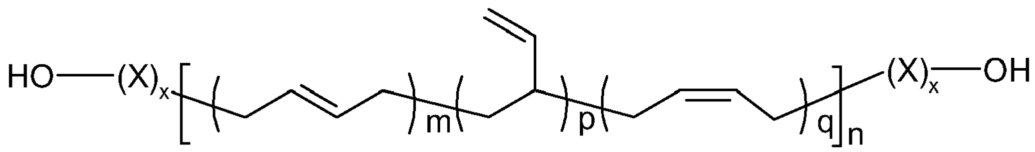

avec
m, n et p étant des fractions molaires comprises entre 0 et 1, et m+n+p=1;
notamment m compris entre 0,1 et 0,8, voire 0,15 et 0,7; p compris entre 0,1 et 0,8, voire 0,15 et 0,7; et q compris entre 0,05 et 0,5, voire 0,1 et 0,4;
n est un entier compris entre 10 et 100, notamment entre 15 et 50;
x = 0 ou 1, et
X = radical carboné divalent, notamment alkylène, linéaire, cyclique ou ramifié, comprenant 1 à 10 atomes de carbone; notamment méthylène, éthylène, propylène ou isopropylène.
- d'au moins un motif résultant de diisocyanates choisis parmi le méthylènecyclohexanediisocyanate, l'isophorone diisocyanate, le 1,4-butanediisocyanate et le 1,6-hexane-diisocyanate, et leur mélange; de préférence l'isophorone diisocyanate.

**23.** Polyuréthane selon l'une des revendications précédentes, dans lequel :

- les amines formant les motifs cationiques ou cationisables (a1) représentent de 0,1 à 50%, de préférence de 1 à 30%, mieux de 5 à 20% en poids, du poids total du polyuréthanne final; et/ou
- les polyoléfines formant les motifs non ioniques (a2) représentent de 30 à 99% en poids, de préférence de 50 à 90%, mieux de 60 à 80% en poids, du poids total de polyuréthanne final; et/ou
- les composés comprenant au moins deux fonctions isocyanate formant les motifs (b) représentent de 1 à 60% en poids, notamment 5 à 50% en poids, mieux 15 à 35% en poids, du poids total du polyuréthanne final.

**24.** Composition cosmétique ou pharmaceutique comprenant, dans un milieu physiologiquement acceptable, au moins un polyuréthane tel que défini à l'une des revendications précédentes.

**25.** Composition selon la revendication 24, dans laquelle le polyuréthanne est présent en une quantité comprise entre 0,1 et 90% en poids, de préférence entre 1 et 50% en poids, notamment entre 2 et 25% en poids, voire entre 5 et 15% en poids, et mieux entre 6 et 10% en poids, par rapport au poids de la composition.

**26.** Composition selon l'une des revendications 24 à 25, dans laquelle le milieu physiologiquement acceptable comprend au moins un composé choisi parmi l'eau, les alcools, les polyols, les éthers de polyols ou de glycols, les aldéhydes en $C_2$-$C_4$ hydrophiles, les esters, les huiles carbonées, les huiles de silicone, les huiles de silicone fluorées; les gélifiants et/ou épaississants, tels que les polymères, associatifs ou non, anioniques, non ioniques ou cationiques; les tensioactifs anioniques, non ioniques, cationiques et/ou amphotères; les agents propénétrants, les émulsionnants, les parfums, les conservateurs, les charges, les filtres solaires; les silicones; les matières colorantes, les protéines, les vitamines, les provitamines; les polymères, fixants ou non fixants, anioniques, non ioniques, cationiques ou amphotères; les agents hydratants, les émollients, les agents adoucissants; les huiles minérales, végétales ou synthétiques; les actifs hydrophiles ou lipophiles comme les céramides et les pseudocéramides; les agents antimousse, les agents antiperspirants, les agents anti-radicaux libres, les agents bactéricides et les agents anti-pelliculaires; ainsi que leurs mélanges.

**27.** Composition selon l'une des revendications 24 à 26, se présentant sous la forme d'un produit de soin, de nettoyage et/ou de maquillage de la peau du corps ou du visage, des lèvres, des cils, des ongles et des cheveux; d'un produit solaire ou autobronzant; d'un produit d'hygiène corporelle; d'un produit capillaire, notamment de soin, de nettoyage, de coiffage ou de coloration des cheveux.

**28.** Composition selon l'une des revendications 24 à 27, se présentant sous la forme d'une composition capillaire, notamment pour le maintien de la coiffure ou la mise en forme des cheveux.

**29.** Composition selon l'une des revendications 24 à 27, se présentant sous la forme d'une composition capillaire de type laque pour cheveux, et dans laquelle le polyuréthane est présent à une teneur comprise entre 0,1 et 25% en poids, notamment entre 1 et 20% en poids, mieux entre 2 et 15% en poids, voire entre 4 et 8% en poids, par rapport au poids de la composition.

**30.** Composition selon l'une des revendications 24 à 27, se présentant sous la forme d'une composition de coiffage dans laquelle le polyuréthane est présent à une teneur de 0,5 à 20% en poids, notamment entre 1 et 15% en poids, mieux entre 2 et 10% en poids, voire entre 5 et 8% en poids, par rapport au poids de la composition.

**31.** Composition selon l'une des revendications 24 à 27, se présentant sous la forme d'une composition capillaire de type shampooing dans laquelle le polyuréthane est présent à une teneur de 0,1 à 20% en poids, notamment 0,5 à 15% en poids, mieux entre 1 et 10% en poids, voire entre 2 et 5% en poids, par rapport au poids de la composition.

**32.** Procédé de traitement cosmétique, notamment de maquillage, de soin, de nettoyage, de coloration, de mise en forme des matières kératiniques, notamment de la peau du corps ou du visage, des ongles, des cheveux, des poils et/ou des cils, comprenant l'application sur lesdites matières d'une composition cosmétique telle que définie à l'une des revendications 24 à 31.

**33.** Procédé de traitement cosmétique pour la mise en forme et/ou le maintien des cheveux, comprenant l'application d'une composition cosmétique telle que définie à l'une des revendications 24 à 31 sur lesdits cheveux; éventuellement suivie d'une étape de rinçage.

Office européen des brevets

# RAPPORT DE RECHERCHE EUROPEENNE

Numéro de la demande
EP 07 10 1734

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| P,X | EP 1 645 579 A (OREAL [FR]) 12 avril 2006 (2006-04-12) * alinéa [0007] - alinéa [0033]; revendications 1,3,10,11,23,27-32 * | 1-33 | INV. C08G18/08 C08G18/69 A61K8/87 A61Q5/06 |
| X | WO 02/36653 A2 (CROMPTON CORP [US]) 10 mai 2002 (2002-05-10) * revendications 1-22; exemple 1 * | 1-31 | |
| X<br>Y | WO 02/09655 A2 (OREAL [FR]) 7 février 2002 (2002-02-07) * revendications 1,2,7,8,12,13,24; exemples 1-3 * | 1-33<br>1-33 | |
| Y | EP 1 543 819 A (OREAL [FR]) 22 juin 2005 (2005-06-22) * alinéa [0009] - alinéa [0049]; revendications 1-29 * | 1-33 | |
| Y | EP 1 329 470 A (OREAL [FR]) 23 juillet 2003 (2003-07-23) * alinéa [0015]; revendication 1 * | 1-33 | DOMAINES TECHNIQUES RECHERCHES (IPC)<br>C08G<br>A61K<br>A61Q |

Le présent rapport a été établi pour toutes les revendications

1

| Lieu de la recherche | Date d'achèvement de la recherche | Examinateur |
|---|---|---|
| Munich | 2 mai 2007 | Scheuer, Sylvie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 07 10 1734

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-05-2007

| Document brevet cité au rapport de recherche | | Date de publication | Membre(s) de la famille de brevet(s) | | | Date de publication |
|---|---|---|---|---|---|---|
| EP 1645579 | A | 12-04-2006 | BR | PI0506007 | A | 06-06-2006 |
| | | | CN | 1775826 | A | 24-05-2006 |
| | | | FR | 2875503 | A1 | 24-03-2006 |
| | | | JP | 2006104463 | A | 20-04-2006 |
| | | | KR | 20060051504 | A | 19-05-2006 |
| WO 0236653 | A2 | 10-05-2002 | US | 6339125 | B1 | 15-01-2002 |
| WO 0209655 | A2 | 07-02-2002 | AU | 8238701 | A | 13-02-2002 |
| | | | EP | 1307177 | A2 | 07-05-2003 |
| | | | JP | 2004515466 | T | 27-05-2004 |
| | | | US | 2004141942 | A1 | 22-07-2004 |
| | | | US | 6613314 | B1 | 02-09-2003 |
| EP 1543819 | A | 22-06-2005 | BR | 0405700 | A | 20-12-2005 |
| | | | CA | 2490699 | A1 | 19-06-2005 |
| | | | CN | 1650840 | A | 10-08-2005 |
| | | | FR | 2863884 | A1 | 24-06-2005 |
| | | | JP | 2005220127 | A | 18-08-2005 |
| | | | MX | PA04012643 | A | 01-07-2005 |
| EP 1329470 | A | 23-07-2003 | BR | 0300096 | A | 02-09-2003 |
| | | | CA | 2414425 | A1 | 21-07-2003 |
| | | | CN | 1434065 | A | 06-08-2003 |
| | | | FR | 2834992 | A1 | 25-07-2003 |
| | | | JP | 2003226731 | A | 12-08-2003 |
| | | | MX | PA03000573 | A | 14-02-2005 |
| | | | PL | 358343 | A1 | 28-07-2003 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9413724 A
- EP 619111 A
- WO 0232978 A
- US 3388087 A